# EUROPÄISCHE PATENTSCHRIFT

(11) **EP 0 565 072 B1**
(45) Veröffentlichungstag und Bekanntmachung des Hinweises auf die Patenterteilung: **26.03.1997**
(21) Anmeldenummer: 93105731.9
(22) Anmeldetag: 07.04.1993
(51) Int. Cl.: A61K 7/04, A61K 7/043

(54) **Nagellack zur Behandlung von Onychomykosen**
Nail lacquer for the treatment of onychomycoses
Vernis à ongles pour le traitement des onychomycoses

(30) Priorität: 10.04.1992 DE 4212105
(43) Veröffentlichungstag der Anmeldung: 13.10.1993
(73) Patentinhaber: Esparma Pharmazeutische Fabrik GmbH, D-39114 Magdeburg (DE)
(72) Erfinder: Wohlrab, Wolfgang, Prof. Dr., D-06128 Halle (DE); Wellner, Katrin, Dr., D-06108 Halle (DE)
(74) Vertreter: Pfenning, Meinig & Partner

(56) Entgegenhaltungen:
- EP-A- 0 204 230
- EP-A- 0 226 984
- GB-A- 2 202 743
- CHEMICAL ABSTRACTS, vol. 96, no. 20, 1982, Columbus, Ohio, US; abstract no. 168622k, G. STUETTGEN 'bioavailability, skin and nail penetration of topically applied antimycotics' Seite 394 ;
- PARFUMS, COSMETIQUES, AROMES Bd. 79, 1988, FRANCE Seiten 47 - 61 H. DJELASSI 'l'ongle: griffe ou ornement'

## Beschreibung

### Gegenstand der Erfindung

Die Erfindung betrifft einen Nagellack zur Behandlung von Onychomykosen, enthaltend einen Filmbildner, einen antimykotisch wirksamen Stoff oder Stoffgemisch und Harnstoff, wobei sowohl der antimykotisch wirksame Stoff als auch der Harnstoff aus dem Lack freigesetzt werden.

### Stand der Technik

Die Therapie der Onychomykosen ist anerkanntermaßen schwierig und langwierig. Eine orale Therapie mit Antimykotika erfordert monatelange Behandlung, ist nur in etwa 50 % der Fälle erfolgreich und muß streng auf Nebenwirkungen überwacht werden.
Als zusätzliche lokale Maßnahme wird die mechanische Ablösung betroffener Nagelareale und eine lokale Behandlung mit antimykotikahaltigen Arzneizubereitungen praktiziert. Eine Extraktion der Nägel, insbesondere bei multiplem Befall, ist für die Patienten häufig unzumutbar (vgl. hierzu H. Mensing und H. Walther, Dermatologie in der täglichen Praxis, Seiten 239 ff, Gustav-Fischer-Verlag Stuttgart-New York, 1989).
Die topische Anwendung entsprechender Präparate ist allerdings aufgrund unzureichender Penetration durch das Nagel-Keratin uneffektiv. Die Empfehlung, kleine Löcher in den Nagel zu bohren, um die Fungizide in tiefere Schichten zu bringen, erscheint in diesem Zusammenhang recht aufwendig (J. Brem, Cutis 27, Seite 69, 1981, Effective topical method for therapy of onychomycosis).
Seit längerer Zeit werden Methoden angewandt, die sich einer speziellen Galenik antimykotikahaltiger Arzneipräparate bedienen. Die Behandlung der Nägel mit einer hochprozentigen Lösung von Harnstoff und Natriummetabisulfid führt zu einer Spaltung der Disulfid- und Wasserstoffbrücken des Keratins, wodurch eine bessere Penetration der Fungizide erreicht wird (vgl. hierzu J.E. Barlow and F.W. Chattaway, Lancet II, Seite 1269, 1955; V. Ramesh et al., Int. J. of Dermatology 22, Seite 148, 1983).
Ebenfalls bewährt in der Anwendung hat sich eine Kaliumjodid-haltige Salbe auf Lanolinbasis. Die auf Rekristallisationserscheinungen zurückzuführende Grobkörnigkeit der Zubereitung und die durch freiwerdendes Jod auftretenden Verfärbungen stellen jedoch echte Mängel dar. (H. E. Kleine-Watrop, Dermatol. Mon. schr. 165, Seite 137, (1979).
In jüngerer Zeit ist man dazu übergegangen, Salben mit einem Harnstoffgehalt von bis zu 40 Gew.-% zur Onycholyse einzusetzen. Gesundes Nagelmaterial wird dabei nicht angegriffen. Zubereitungen von 30 bis 40 Gew.-% Harnstoff in Wollwachsalkoholsalbe bzw. ähnliche Grundlagen werden häufig von Dermatologen formuliert und rezeptiert. Daneben stehen auch bereits entsprechende Fertigpräparate zur Verfügung. Die Salben werden auf den Nagel appliziert und mit einem Pflaster abgedeckt, wobei das erweichte Nagelmaterial nach etwa 24 Stunden mit einem Schaber entfernt wird.

Eine Reihe von Patentanmeldungen beschreibt Nagellacke, die lokal begrenzt auftragbar sind.

JP-A 89-99 159 und JP-A 87-266 059 beanspruchen Nagellacke, die ein Antimykotikum, Polyvinylacetat als Filmbildner und flüchtige Lösungsmittelbestandteile enthalten. Der sich auf dem Nagel ausbildende Film wird nach 6 Stunden abgezogen, wobei bei wiederholter Behandlung bei 3 Patienten nach 3 Monaten die vollständige Heilung einer Nagel-Trichophytose erreicht werden konnte.
DE-OS 35 44 983 beschreibt Nagelbeschichtungen, die einen wasserunlöslichen Filmbildner und ein Antimykotikum enthalten.
In der PCT-Anmeldung WO 87 02 580 werden Nagellacke, die einen hydrophilen Filmbildner und ein Antimykotikum mit einem Molekulargewicht < 550 Dalton enthalten, beschrieben. Nach Auftrag des Nagellacks bildet sich innerhalb von 3 Minuten ein nicht klebender, transparenter Film. Bei einer zweimaligen Behandlung pro Woche konnte eine Onychomykose-Infektion innerhalb von 8 Monaten geheilt werden.
EP-A 298 271 beansprucht Nagellacke mit einem Gehalt an mindestens einem wasserunlöslichen Filmbildner und mindestens einem antimykotisch wirksamen Stoff aus der Gruppe Tioconazol, Econazol, Oxiconazol, Miconazol, Tolnaftat und Naftifinhydrochlorid. Die Nagellacke werden vorzugsweise als medizinische Nagellacke angewandt, die eine wirksame Menge des antimykotischen Wirkstoffs enthalten, die zur Abtötung der Nagelmykosen verursachenden Dermatophyten ausreicht.
In der PCT-Anmeldung WP 88 06 884 wird eine topisch aufzutragende Formulierung zur Behandlung von Nagelmykose beschrieben, die aus einem Antimykotikum, einem Antiseptikum und einem Filmbildner bestehen. Eine 28 Wochen lange Behandlung von Nagelmykose war von befriedigendem Erfolg.

Die GB 2 202 743 offenbart einen Nagellack, der Miconazol-Nitrate oder Econazol-Nitrate, gelöst in einer Mischung aus Wasser und Harnstoff sowie einem Lösungsvermittler, enthält. Wesentlich bei diesem Nagellack ist offensichtlich, daß die Anteile Harnstoff nicht über die im Nagellack enthaltenen Wasseranteile hinausgehen. Der Wasseranteil ist somit in dem vorliegenden Nagellack von entscheidender Bedeutung.

### Aufgabe und Lösung

Trotz der guten Beurteilung des Therapieeffekts harnstoffhaltiger Salben ist die Behandlung noch mit Unannehmlichkeiten für den Patienten verbunden, welche sich durch eine galenische Verabreichung in Form eines Nagellacks reduzieren lassen. Durch die Möglichkeit, den Lack lokal begrenzt auf den Nagel auftragen zu können, wird eine Reizung der umgebenden Hautareale ausgeschlossen. Zudem erübrigt Sich die Anwendung eines Pflasters, da der Lack einen festen Film auf dem zu behandelnden Nagel bildet.
Auf der anderen Seite steht die Frage, ob und wie schnell aus einem solchen Film der antimykotische Wirkstoff auch an den Nagel freigegeben wird und in welchem Zeitraum der Wirkstoff die onychomykotisch befallenen Stellen im Nagel erreicht.
Aus dem diskutierten Stand der Technik und den oben genannten Anforderungen resultiert die Aufgabe, einen Nagellack zur Behandlung der Onychomykose zu entwickeln, der lokal als leicht entfernbarer Film auf die befallenen Nagelteile aufgetragen werden kann, eine gute Wirkstoff-Liberation aufweist und eine gute Diffusion des antimykotischen Wirkstoffs in die onychomykotisch befallenen Bezirke des Nagels gewährleistet, ohne das gesunde Nagelmaterial anzugreifen.
Es wurde gefunden, daß Nagellacke, die einen Filmbildner, vorzugsweise ein Cellulosederivat, einen antimykotisch wirksamen Stoff, vorzugsweise Clotrimazol, Harnstoff und gegebenenfalls Weichmacher in einem Lösungsmittelgemisch enthalten, das aus einer Komponente A besteht, die den Filmbildner anquillt, und einer Komponente B, die die Wirkstoffe Harnstoff und Antimykotikum löst, besteht, die o.g. Aufgabenstellung in hervorragender Weise löst.

### Durchführung der Erfindung

### Die Filmbildner

Die Filmbildner sind Polymerisate, die beim Verdunsten des Lösungsmittels zusammenhängende Filme bilden, die für den antimykotischen Wirkstoff und den Harnstoff permeabel sind.
So können beispielsweise Polymerdispersionen verwendet werden, deren Glasübergangstemperatur beim Trocknen überschritten wird und die dann einen kontinuierlichen Polymerfilm ausbilden. Beschichtet man eine Substratoberfläche, wie beispielsweise einen Fingernagel, mit einer solchen Polymerdispersion und trocknet diese durch Verdunsten des Lösungsmittels, so resultiert eine filmbeschichtete Oberfläche (vgl. hierzu Kirk-Othmer, Encyclopedia of Chemical Technology, 3rd. Ed. Vol. 14, Seite 90, J. Wiley, New York. 1981).
Solche Polymerdispersionen können aufgebaut sein aus Polyvinylacetat, Mischpolymerisaten aus Vinylacetat und Acrylsäure, Mischpolymerisaten aus (Meth)acrylsäure und (Meth)acrylsäureestern, Polyvinylacetat oder Polyvinylbutyral.

Als Filmbildner werden bevorzugt Cellulosederivate, wie Celluloseacetatphthalat, Celluloseacetatbutyrat, Celluloseacetatpropionat, Cellulosenitrat, Cellulosesulfat, Ethylcellulose sowie Celluloseacetat verwendet. Zur Herstellung und den Filmbildungseigenschaften dieser Cellulosederivate vgl. Kirk-Othmer, Encyclopedia of Chemical Technology, 3rd. Ed. Vol. 5, Seiten 70 bis 163, J. Wiley, New York, 1979.

### Die Kombination aus Antimykotium und Harnstoff

Die erfindungsgemäß verwendeten Antimykotika sind vorzugsweise Imidazolderivate wie: 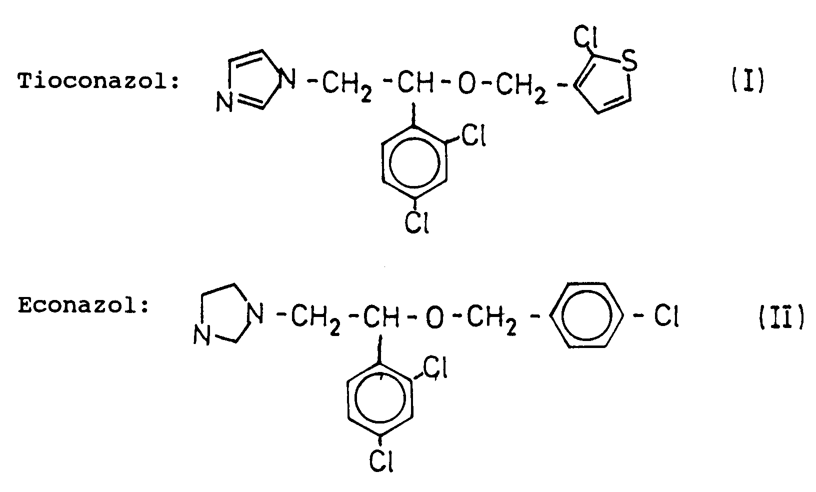 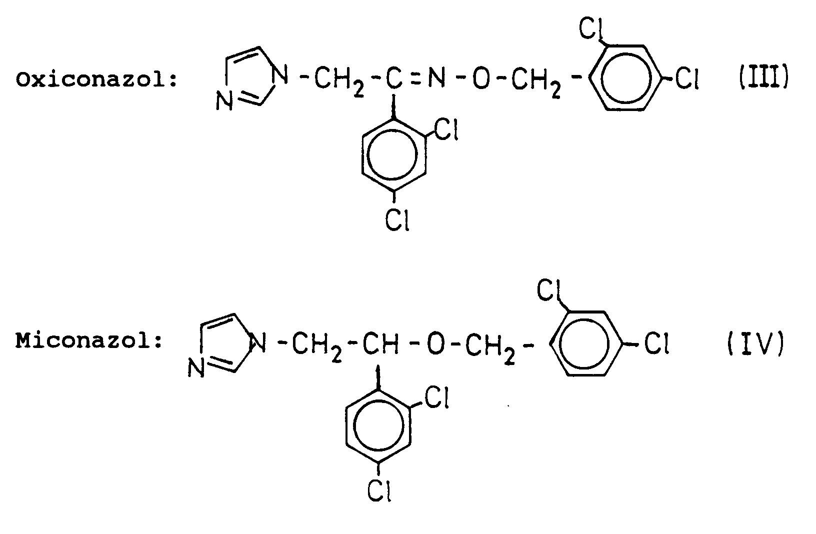 und besonders bevorzugt:

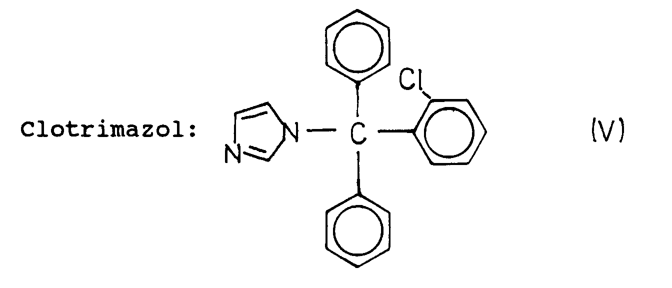

Der in Verbindung mit dem Antimykotikum eingesetzte Harnstoff ist für seine keratolytische Wirkung bekannt (EP-A 298 271). Deshalb war zu erwarten, daß bei den erfindungsgemäßen Anteilen (bezogen auf die nichtflüchtigen Bestandteile des Nagellacks) von 15 bis 60 Gew.-%, vorzugsweise 20 bis 50 Gew.-% Harnstoff in Verbindung mit 5 bis 30 Gew.-% Antimykotikum 1.0 und zu 100 Gew % ergänzte Anseite an Filmbildner eine starke Keratolyse stattfinden würde, die gleichermaßen gesunde wie auch onychomykotisch befallene Bereiche des Nagels aufweicht. Zur keratolytischen Wirkung von Harnstoff und zur optimalen Entfaltung von Eigenschaften von Harnstoff/Wirkstoff-Kombinationen vgl. W. Wohlrab, Dermatol. Monatsschrift 167, Seiten 188 - 191 (1981). Dort wird darauf hingewiesen, daß eine verbesserte Wirksamkeit extern applizierter Arzneimittel durch Harnstoffzusatz nur durch den additiven Effekt mehrerer Faktoren zustande kommt und für den Fachmann damit nicht naheliegend ist. Überraschenderweise wird der Harnstoff und das Antimykotikum vom gebildeten Film auf dem Nagel derart dosiert abgegeben, daß die gesunden Stellen des Nagels keiner nennenswerten Keratolyse unterworfen sind. Damit wird eine lokal gezielte Behandlung von Onychomykosen möglich, bei maximaler Erhaltung der gesunden Nagelsubstanz.

### Das Lösungsmittel für Filmbildner, Antimykotikum und Harnstoff

Die Lösungsmittel für den erfindungsgemäßen Lack, die in Anteilen von mindestens 50 Gew.-%, bezogen auf die Lackformulierung, bevorzugt von mindestens 70 Gew.-%, besonders bevorzugt von mindestens 80 Gew.-% anwesend sind, sind ausgewählt aus Alkohole, Ketone, Ether, aromatische Kohlenwasserstoffe, wie beispielsweise Toluol, oder Ester, wie Essigsäureethylester sein. Bevorzugt sind Lösungsmittelkombinationen, deren Komponenten den Filmbildner gut anquellen und die Wirkstoffe Antimykotikum und Harnstoff gut lösen. Besonders bevorzugt sind herbei Mischungen aus Ketonen und Alkoholen, wie beispielsweise Aceton/Ethanol-Mischungen für den Filmbildner Celluloseacetat und die Wirkstoffe Harnstoff als Keratolytikum und Clotrimazol als Antimykotikum.
Gleichzeitig muß das Lösungsmittel, oder vorzugsweise das Lösungsmittelgemisch, einen ausreichenden Verdunstungsgrad aufweisen, um Trocknungszeiten des Nagellacks im Minutenbereich zu gewährleisten. Neben der Trocknungszeit sind von Bedeutung: Verlauf des Nagellacks, Filmbildegeschwindigkeit, Viskosität und weitere wichtige Lackeigenschaften. Um eine gleichmäßige Verdunstung zu erreichen, besteht das vorzugsweise eingesetzte Lösungsmittelgemisch aus einer Mischung von Lösungsmitteln unterschiedlicher Siedepunkte. Geeigneterweise werden hierfür Lösungsmittel mit Siedepunkten zwischen 40 und 150 Grad C gewählt.
Das Gemisch aus Lösungsmittel, Filmbildner, Harnstoff und Antimykotikum enthält vorzugsweise noch Weichmacher, wie beispielsweise Triacetin, Propylenglykol, Ricinusöl, Campher oder Phthalate, besonders bevorzugt Dibutylphthalat. Die Weichmacher haben Einfluß auf die Liberation des Harnstoffs und des antimykotischen Wirkstoffs und sind darauf individuell abzustimmen. Die Kombination aus Filmbildner, Harnstoff, Antimykotikum und gegebenenfalls Weichmacher werden als nichtflüchtige Bestandteile bezeichnet.
Daneben sind weitere in der Kosmetik gebräuchliche michtflüchtige Zusätze zum Nagellack wie Farbpigmente, Perlglanzpigmente, Kolloidstabilisatoren, UV-Stabilisatoren und/oder antibakteriell wirksame Substanzen möglich.

Die Herstellung des Nagellacks erfolgt nach den bekannten Verfahren der Lackformulierung wie beispielsweise in Kirk-Othmer, Encyclopedia of Chemical Technology, 3rd. Ed., Vol. 6, Seiten 427 bis 445, J. Wiley, 1979 dargestellt.

In einer bevorzugten Ausführungsform wird der Filmbildner zunächst mit einer Komponente A des Lösungsmittelgemischs angequollen, bevor in einem weiteren Schritt die in einer Komponente B des Lösungsmittelgemischs gelösten Wirkstoffe Antimykotikum, Harnstoff und gegebenenfalls Weichmacher und/oder weitere Additive zugegeben werden. Besonders bevorzugt werden Ketone und Ester als Komponente A verwendet, im Falle des Filmbildners Celluloseacetat ganz besonders bevorzugt Aceton, sowie Alkohole als Komponente B, im Falle des Antimykotikums Clotrimazol und des Harnstoffs ganz besonders bevorzugt Ethanol.

### Vorteilhafte Wirkungen der Erfindung

Der erfindungsgemäße Nagellack besitzt onycholytische und antimykotische Wirkung, wobei die Wirkstoffe Harnstoff und Antimykotikum derart vom Lack freigesetzt werden, daß die gesunden Stellen des Nagels keiner nennenswerten Keratolyse unterworfen sind und damit eine lokal gezielte Behandlung von Onychomykosen bei maximaler Erhaltung der Nagelsubstanz möglich wird.
Die Lackpräparationen lassen sich gut auftragen und trocknen schnell zu einem glatten, glänzenden Film, welcher bei der Harnstoff enthaltenden Rezeptur aufgrund des hohen Harnstoff- und Feststoffanteils weiß gefärbt ist. Die lokal gezielte Auftragung des Nagellacks ist insbesondere günstig, da die hohen Harnstoffanteile bei ungezielter Auftragung, beispielsweise durch Salben, zu einem keratolytischen Angriff auf die den Nagel umgebende Haut führen würde.

Bei Liberationsuntersuchungen konnte nachgewiesen werden, daß sowohl der Harnstoff als auch das Antimykotikum in der gewünschten Dosierung aus dem Lack freigesetzt werden. Der gegebenenfalls zugesetzte Weichmacher fördert die Liberation von Harnstoff und Antimykotikum, wirkt sich jedoch bei hohen Anteilen negativ auf die Lackeigenschaften aus: der Lack wird klebrig.

Von Vorteil ist desweiteren, daß die Wirkstoffe Harnstoff und Antimykotikum nicht systemisch, wie beispielsweise oral oder intravenös, appliziert werden müssen.

Die folgenden Beispiele dienen zur Erläuterung der Erfindung.

### BEISPIELE

### Beispiel 1:

### Lackpräparation Die Zusammensetzung des Nagellacks lautet:

0,5 g Celluloseacetat
0,15 g Weichmacher
0,28 g Harnstoff
0,10 g Clotrimazol
als Feststoff in einem Lösungsmittel-Gemisch von:
3,10 g Ethanol (90 Vol.-%)
5,90 g Aceton

Zunächst wurde das Celluloseacetat in Aceton angequollen. Danach erfolgte die Zugabe der in Ethanol gelösten Wirkstoffe Harnstoff und Clotrimazol nebst Weichmacher.

### Beispiel 2:

### Wahl des geeigneten Lösungsmittelsystems

Besondere Anforderungen stellen sich bei der Wahl eines geeigneten Lösungsmittelsystems: Es soll eine ausreichende Quellung des Filmbildners gewährleisten, ein ausreichendes Lösungsvermögen für die einzusetzenden Wirkstoffe Harnstoff und Antimykotikum aufweisen und gleichzeitig für eine vertretbare Trocknungsdauer des Nagellacks verantwortlich sein. Auswahl und Optimierung des Lösungsmittels wurde über die Ermittlung des Lösevermögens für Harnstoff und des Verdunstungsgrads (vgl. hierzu K. Rothemann, Das große Rezeptbuch der Haut- und Körperpflegemittel, Dr. Alfred Hüthig Verlag, Heidelberg, 1969) vorgenommen. Als optimal erwies sich eine Kombination von Aceton und Ethanol (90 Vol.-%, 10 Vol.-% H₂O) im Mischungsverhältnissen von 70 bis 50 Gew.-% Aceton und 30 bis 50 Gew.-% Ethanol.

### Beispiel 3:

### Auswahl des Weichmachers und Liberation der Wirkstoffe Harnstoff und Clotrimazol

Als Weichmacher wurden Triacetin, Propylenglykol, Ricinusöl und Dibutylphthalat getestet. Hinsichtlich ihrer äußeren Beschaffenheit konnten alle Lacke als gut beurteilt werden, jedoch zeigten sich erhebliche Unterschiede in der Harnstoff- und Clotrimazolliberation (Tabellen 3a und 3b).

**Tabelle 1a**

| Harnstoffliberation aus Lackpräparationen in Abhängigkeit von der Art des Weichmachers | | | |
|---|---|---|---|
| Weichmacher | Harnstoffliberation | | |
| | % | ug | mmolar |
| Triacetin | 0,76 ± 0,05 | 11,4 | 21,39 |
| Propylenglykol | 0,40 ± 0,17 | 6,0 | 11,26 |
| Ricinusöl | 0,20 ± 0,08 | 3,0 | 5,63 |
| Dibutylphthalat | 4,42 ± 0,91 | 66,3 | 124,39 |

**Tabelle 1b**

| Clotrimazolliberation aus Lackpräparationen in Abhängigkeit vom eingesetzten Weichmacher | | | | | | |
|---|---|---|---|---|---|---|
| Weichmacher | Clotrimazolliberation | | | | | |
| | 1. | 2. | 3. | gesamt | | |
| | Membran | | | % | ug | mmolar |
| Triacetin | 4,55 ±0,82 | 2,90 ±0,21 | 2,58 ±0,10 | 10,03 ± 0,81 | 5,0 | 1,64 |
| Propylengglykol | 4,10 ±0,54 | 3,70 ±0,29 | 2,46 ±0,73 | 10,26 ± 0,62 | 5,1 | 1,68 |
| Ricinusöl | 13,96 ± 0.88 | 6,42 ±1,15 | 4,70 ±1,21 | 25,10 ± 1,79 | 12,6 | 4,10 |
| Dibutylphthalat | 5,95 ±0,19 | 5,28 ±0,15 | 3,95 ±0,58 | 15,18 ± 0,67 | 7,6 | 2,48 |

Die Untersuchungen zur Harnstoff- bzw. Clotrimazolfreigabe erfolgten am Multimembranmodell (vgl. hierzu R. Neubet, W. Wohlrab, Acta Pharm. Technol. 36, 197, 1990) unter modifizierten Bedingungen. Zum Einsatz kamen Dodecanol-Collodium-Membranen bei einer Versuchsdauer von 14 Stunden. Wegen des in der Lack-Formulierung enthaltenen Lösungsmittels konnte der Lack nicht direkt auf die Membran aufgetragen werden, sondern wurde auf eine Aluminiumfolie in einem Feld von 2 cm mal 2 cm aufgetragen und nach dem vollständigen Trocknen mit der oben genannten Membran in einer Zelle fest verpreßt. Die aufgetragene Lackmenge betrug etwa 5 mg. Da hierbei Schwankungen unvermeidlich sind, erfolgte die Auswertung über die prozentual an die Membran freigegebene Wirkstoffmenge entsprechend der jeweiligen Lackeinwaage unter Angabe der Standardabweichung vom mindestens 4 Parallelversuchen (vgl. Tabellen 1a und 1b). Die Angaben in ug bzw. molarer Konzentration ergeben sich durch Umrechnung der Mittelwerte auf die in genau 5 mg Lackmenge enthaltenen Harnstoff- bzw. Antimykotikumanteile.

### Harnstoff-Analytik

Der Harnstoff wurde durch 30-minütiges Schütteln mit 4,0 ml Wasser aus der Membran extrahiert. 2,0 ml Probe wurden mit 1,0 ml einer 0,1 %-igen wäßrigen Diacetylmonoximlösung, 0,5 ml einer Lösung von N-(1-Naphthyl)ethylendiammoniumdichlorid (0,005 mol/l) mit 0,05 % Natriumhydrogencarbonat sowie 0,5 ml konzentrierter Schwefelsäure versetzt. Nach 10-minütiger Reaktionszeit im kochenden Wasserbad erfolgte sofort ein Zusatz von 0,25 ml 1 %-iger Kaliumpersulfatlösung. Nach Farbentwicklung innerhalb von 10 - 15 min wurden die Proben spektralphotometrisch bei 564 nm vermessen.

### Clotrimazol-Analytik

Nach Extraktion des Arzneistoffs aus der Membran während 30-minütigen Schüttelns mit 4,0 ml Cloroform folgte Zugabe von 5,0 ml Farbreagens. Hierbei handelt es sich um eine Lösung von 100 mg Methylorange in 100 ml Borsäure (0,5 mol/l), frisch bereitet und filtriert. Nach 5-minütigem Schütteln wurden 2,0 ml der organischen Phase mit 300 ul Schwefelsäurereagens (2,0 ml konzentrierte Schwefelsäure + 100,0 ml Ethanol) versetzt und spektralphotometrisch bei 520 nm vermessen.

### Ergebnisse

Aus den unterschiedlichen physikochemischen Eigenschaften von Harnstoff und Clotrimazol resultiert ein unterschiedliches Verteilungs- und Liberationsverhalten.
Gegenüber den anderen Weichmachern bewirkt Dibutylphthalat eine 6- bis 22-fache Harnstoffliberation (Tab. 3a). Bezüglich der Antimykotikumfreisetzung erweist sich Ricinusöl als günstigste Variante (Tab. 3b).
Im Hinblick auf einen optimalen Therapieeffekt, der ganz entscheidend von einer erfolgreichen Onycholyse bestimmt wird, wurde die Rezeptur mit Dibutylphthalat als Weichmacher ausgewählt.
Nach erfolgreicher Entfernung des erkrankten Nagelmaterials bietet sich dann die Möglichkeit, für den verbleibenden, noch gesunden Nagel, einen harnstofffreien Lack mit optimierter Antimykotikumkonzentration anzubieten.

## Patentansprüche

1. Nagellack zur Behandlung von Onychomykosen, enthaltend einen polymeren Filmbildner, mindestens einen antimykotisch wirksamen Stoff und Harnstoff,
dadurch **gekennzeichnet**, daß er 5 - 30 Gew.-% antimykotischen Wirkstoff, 15 - 60 Gew.-% Harnstoff und zu 100 Gew.-% ergänzte Anteile an Filmbildnern, bezogen auf die nichtflüchtigen Bestandteile, enthält, und bezogen auf die Lackformulierung insgesamt, mindestens 50 Gew.-% Lösungsmittel, ausgewählt aus Alkoholen, Ketonen, Ethern, aromatischen Kohlenwasserstoffen oder deren Mischungen, wobei der Nagellack nach topischer Anwendung und Trocknung sowohl den antimykotisch wirksamen Stoff als auch den Harnstoff aus dem Lack freisetzt.

2. Nagellack nach Anspruch 1,
dadurch gekennzeichnet, daß der Filmbildner ein Zellulosederivat ist.

3. Nagellack nach Anspruch 1 oder 2,
dadurch gekennzeichnet, daß der antimykotisch wirksame Stoff Clotrimazol ist.

4. Nagellack nach mindestens einem der Ansprüche 1 bis 3,
dadurch gekennzeichnet, daß er zusätzlich 5 - 30 Gew.-%, bezogen auf die nichtflüchtigen Bestandteile im Filmbildner, eines Weichmachers enthält, der ausgewählt ist aus der Gruppe Phthalsäureester, Glykole oder Rizinusöl.

5. Nagellack nach mindestens einem der Ansprüche 1 bis 4,
dadurch gekennzeichnet, daß er 10 - 20 Gew.-% antimykotischen Wirkstoff enthält.

6. Nagellack nach mindestens einem der Ansprüche 1 bis 5,
dadurch gekennzeichnet, daß er 20 - 50 Gew.-% Harnstoff enthält.

7. Nagellack nach mindestens einem der Ansprüche 1 bis 6,
dadurch gekennzeichnet, daß er mindestens 80 Gew.-% Lösungsmittel enthält.

8. Nagellack nach mindestens einem der Ansprüche 1 bis 7,
dadurch gekennzeichnet, daß das Lösungsmittel ein Gemisch aus Ketonen und Alkoholen ist.

9. Verfahren zur Herstellung eines Nagellacks nach mindestens einem der Ansprüche 1 bis 8,
dadurch gekennzeichnet, daß der Filmbildner zunächst in einem ersten Lösungsmittel bzw. Lösungsmittelgemisch A anquillt, und daß dann die in einem zweiten Lösungs- bzw Lösungsmittelgemisch B gelösten Bestandteile Antimykotikum und Harnstoff zugesetzt werden.

10. Verfahren nach Anspruch 9,
dadurch gekennzeichnet, daß als Lösungsmittel bzw. Lösungsmittelgemisch A Ketone und/oder Ester eingesetzt werden.

11. Verfahren nach Anspruch 10 oder 11,
dadurch gekennzeichnet, daß als Lösungsmittel bzw. Lösungsmittelgemisch B Alkohole eingesetzt werden.

## Claims

1. Nail lacquer for the treatment of onychomycoses, containing a polymeric film-forming agent, at least one antimycotic active substance and urea,
characterised in that it contains 5-30% by weight of antimycotic active agent, 15-60% by weight of urea and proportions of film-forming agents supplemented to 100% by weight, calculated on the basis of the non-volatile constituents, selected from alcohols, ketones, ethers, aromatic hydrocarbons or mixtures thereof, and at least 50% by weight of solvent, calculated on the basis of the lacquer formulation overall, whereby the nail lacquer releases both the antimycotic active substance and the urea from the lacquer after local application and drying.

2. Nail lacquer according to Claim 1, characterised in that the film-forming agent is a cellulose derivative.

3. Nail lacquer according to Claim 1 or 2, characterised in that the antimycotic active substance is Clotrimazole.

4. Nail lacquer according to one of Claims 1 to 3, characterised in that it additionally contains 5-30% by weight, calculated on the basis of the non-volatile constituents in the film-forming agent, of a softener, which is selected from the group comprising phthalic esters, glycols or castor oil.

5. Nail lacquer according to at least one of Claims 1 to 4, characterised in that it contains 10-20% by weight of antimycotic active agent.

6. Nail lacquer according to at least one of Claims 1 to 5, characterised in that it contains 20-50% by weight of urea.

7. Nail lacquer according to at least one of Claims 1 to 6, characterised in that it contains at least 80% by weight of solvent.

8. Nail lacquer according to at least one of Claims 1 to 7, characterised in that the solvent is a mixture of ketones and alcohols.

9. Process for the production of a nail lacquer according to at least one of Claims 1 to 8, characterised in that the film-forming agent is firstly expanded in a first solvent or solvent mixture A; and that the constituents dissolved in a second solvent or solvent mixture B are then added to the antimycotic agent and urea.

10. Process according to Claim 9, characterised in that ketones and/or esters are used as solvent or solvent mixture A.

11. Process according to Claim 10 or 11, characterised in that alcohols are used as solvent or solvent mixture B.

## Revendications

1. Vernis à ongles pour le traitement d'onychomycoses, comprenant un agent filmogène polymère, au moins une substance à action antimycosique et de l'urée, caractérisé en ce qu'il contient 5-30% en poids de substance active antimycosique, 15-60% en poids d'urée et des proportions d'agents filmogènes formant le complément à 100% en poids, par rapport aux composants non volatils, et, par rapport à la composition totale de vernis, au moins 50% en poids de solvant, choisi parmi les alcools, cétones, esters hydrocarbures aromatiques ou leurs mélanges, le vernis à ongles libérant, après application locale et séchage, aussi bien la substance à action antimycosique que l'urée, à partir du vernis.

2. Vernis à ongles selon la revendication 1, caractérisé en ce que l'agent filmogène est un dérivé de cellulose.

3. Vernis à angles selon la revendications 1 ou 2, caractérisé en ce que la substance à action antimycosique est le clotrimazole.

4. Vernis à ongles selon au moins l'une des revendications 1 à 3, caractérisé en ce qu'il contient en outre 5-30% en poids, par rapport aux composants non volatils dans l'agent filmogène, d'un plastifiant qui est choisi parmi les esters d'acide phtalique, les glycols et l'huile de ricin.

5. Vernis à ongles selon au moins l'une des revendications 1 à 4, caractérisé en ce qu'il contient 10-20% en poids de substance active antimycosique.

6. Vernis à ongles selon au moins l'une des revendications 1 à 5, caractérisé en ce qu'il contient 20-50% en poids d'urée.

7. Vernis à ongles selon au moins l'une des revendications 1 à 6, caractérisé en ce qu'il contient au moins 80% en poids de solvant.

8. Vernis à ongles selon au moins l'une des revendications 1 à 7, caractérisé en ce que le solvant est un mélange de cétones et d'alcools.

9. Procédé pour la préparation d'un vernis à ongles selon au moins l'une des revendications 1 à 8, caractérisé en ce que l'on fait d'abord gonfler l'agent filmogène dans un premier solvant ou mélange de solvants A, et en ce que l'on ajoute ensuite les composants agent antimycosique et urée dissous dans un second solvant ou mélange de solvants B.

10. Procédé selon la revendication 9, caractérisé en ce que l'on utilise comme solvant ou mélange de solvants A des cétones et/ou des esters.

11. Procédé selon la revendication 9 ou 10, caractérisé en ce que l'on utilise comme solvant ou mélange de solvants B des alcools.
